# EUROPEAN PATENT APPLICATION

(11) **EP 2 653 550 A1**
(43) Date of publication of application: **23.10.2013**
(21) Application number: 11849225.5
(22) Date of filing: 12.12.2011
(51) Int. Cl.: C12P 7/56

(54) **METHOD FOR PRODUCING LACTATE**

(30) Priority: 13.12.2010 JP 2010277026
(71) Applicant: Toray Industries, Inc., Tokyo, 103-8666 (JP)
(72) Inventor: HORIGUCHI, Miyuki, 2488555 Kanagawa (JP); ITO, Masateru, 2488555 Kanagawa (JP); MUNEGUMI, Takeshi, 4558502 Aichi (JP); YAMADA, Katsushige, 2488555 Kanagawa (JP)
(74) Representative: Kador & Partner
(86) International application number: PCT/JP2011/078655
(87) International publication number: WO 2012/081533

(57) **Abstract**

The present invention relates to a method for producing a lactic acid salt, the method comprising the step of subjecting an aqueous lactic acid salt solution comprising a formic acid salt in an amount of not less than 7.0% by weight with respect to the lactic acid salt to crystallization, and recovering the lactic acid salt. By subjecting the aqueous lactic acid salt solution comprising a formic acid salt in an amount of not less than 7.0% by weight with respect to the lactic acid salt to crystallization, supersaturation of the lactic acid salt can be stabilized, and the recovery of the lactic acid salt can be increased thereby.

## Description

### TECHNICAL FIELD

The present invention relates to a method for producing a lactic acid salt by subjecting an aqueous lactic acid salt solution to crystallization.

### BACKGROUND ART

Lactic acid is widely applied not only to uses such as food and pharmaceuticals, but also to industrial uses as monomers for biodegradable plastics (polylactic acid and the like), so that lactic acid is increasingly demanded. Lactic acid is known to be produced by fermentation by microorganisms, wherein the microorganisms convert substrates containing hydrocarbons such as glucose into lactic acid. Lactic acid is divided into optical isomers, the (L)-isomer and the (D)-isomer, based on the conformation of the substituent bound to the carbon at the α position of carbonyl, and, by appropriately selecting the microorganism for microbial fermentation, (L)- or (D)-lactic acid can be selectively produced, or lactic acid as a mixture of the (L)-isomer and the (D)-isomer (racemic body) can be produced.

Since production of lactic acid by microbial fermentation is generally carried out while maintaining a pH appropriate for the microbial fermentation by addition of an alkaline substance to the culture medium, most lactic acid in the culture medium is present as a lactic acid salt. More specifically, the alkaline substance to be added to the culture medium is often calcium hydroxide, and, in such a case, lactic acid produced by the microbial fermentation is present as calcium lactate in the culture medium. Since calcium lactate shows high calcium absorbability, it is drawing attention as a good calcium source in uses for food.

Further, in cases where lactic acid is used as monomers for biodegradable plastics, the lactic acid to be used is preferably free lactic acid obtained by adding an acidic substance (sulfuric acid, for example) to the culture medium after completion of the fermentation, followed by normal purification operations such as membrane separation and/or ion exchanging. However, in this case, highly pure lactic acid is required, so that impurities such as sugars and proteins contained in the culture medium after completion of the fermentation are removed by a method wherein a lactic acid salt is separated as solids by subjecting the culture medium to crystallization before addition of the acidic substance.

As methods for separating a lactic acid salt by subjecting an aqueous lactic acid salt solution to crystallization, methods wherein water is evaporated from an aqueous lactic acid salt solution under heat and reduced pressure to increase the lactic acid salt concentration in the culture medium to the saturation solubility, followed by performing crystallization by decreasing the temperature are known (Patent Documents 1 and 2), and, in the case of a culture medium, a method wherein a fermentation culture medium of a lactic acid-producing yeast is subjected to crystallization and a lactic acid salt is recovered thereafter is known (Patent Document 3). However, for increasing the recovery in the crystallization, the mother liquor after solid-liquid separation again needs to be subjected to concentration under heat and then cooling, so that a large amount of energy is required and the efficiency is low. Therefore, as a method for recovering a lactic acid salt with high energy efficiency, a method was developed wherein an aqueous lactic acid salt solution before the crystallization operation (microbial fermentation culture medium) is passed through a reverse osmosis membrane to remove organic acids other than lactic acid (acetic acid, formic acid and the like) while lactic acid in the culture medium is concentrated (Patent Document 2). However, the recovery of the lactic acid salt was not necessarily sufficient.

### PRIOR ART DOCUMENTS

### [Patent Documents]

[Patent Document 1] JP 60-217897 A
[Patent Document 2] JP 2009-201506 A
[Patent Document 3] JP 2010-57389 A

### SUMMARY OF THE INVENTION

### PROBLEMS TO BE SOLVED BY THE INVENTION

The present invention aims to provide a method for recovering a lactic acid salt with high efficiency in cases where a lactic acid salt is crystallized from an aqueous lactic acid salt solution.

### MEANS FOR SOLVING THE PROBLEMS

As a result of intensive study to solve the above problem, the present inventors discovered that, when an aqueous lactic acid salt solution contains a formic acid salt in an amount larger than a certain level, supersaturation of the lactic acid salt can be stabilized, so that the lactic acid salt can be concentrated to a concentration exceeding the saturation solubility and an effect to increase the recovery of the lactic acid salt is produced in the crystallization operation, thereby completing the present invention.

That is, the present invention is constituted by (1) to (6) below.
(1) A method for producing a lactic acid salt, the method comprising the step of subjecting an aqueous lactic acid salt solution comprising a formic acid salt in an amount of not less than 7.0% by weight with respect to the lactic acid salt to crystallization, and recovering the lactic acid salt.
(2) The method for producing a lactic acid salt according to (1), wherein the aqueous lactic acid salt solution is an aqueous lactic acid salt solution comprising a formic acid salt in an amount of 7.0 to 40.0% by weight with respect to the lactic acid salt.
(3) The method for producing a lactic acid salt according to (1) or (2), wherein the lactic acid salt is calcium lactate or magnesium lactate.
(4) The method for producing a lactic acid salt according to any one of (1) to (3), wherein the lactic acid salt concentration in the aqueous lactic acid salt solution is 10.0 to 30.0% by weight.
(5) The method for producing a lactic acid salt according to any one of (1) to (4), wherein crystallization of the lactic acid salt is carried out at a temperature of not more than 30°C.
(6) The method for producing a lactic acid salt according to any one of (1) to (5), wherein a concentrate obtained by passing the aqueous lactic acid salt solution through a reverse osmosis membrane at 30 to 60°C is subjected to crystallization.

### EFFECT OF THE INVENTION

By the present invention, supersaturation of a lactic acid salt in an aqueous lactic acid salt solution can be stabilized, and the lactic acid salt can be recovered with high efficiency upon crystallization of the lactic acid salt from the aqueous lactic acid salt solution.

### BRIEF DESCRIPTION OF THE DRAWINGS

Fig. 1 shows solubility curves for calcium lactate containing 0, 2.5, 7.2, 14.5 or 25.0% by weight of calcium formate with respect to calcium lactate, which were obtained by 1 hour of incubation.
Fig. 2 shows solubility curves for calcium lactate containing 0, 2.5, 7.2, 14.5 or 25.0% by weight of calcium formate with respect to calcium lactate, which were obtained by 3 hours of incubation.
Fig. 3 shows solubility curves for calcium lactate containing 0, 2.5, 7.2, 14.5 or 25.0% by weight of calcium formate with respect to calcium lactate, which were obtained by 6 hours of incubation.

### BEST MODE FOR CARRYING OUT THE INVENTION

The present invention is described below in more detail.

The present invention is a method for producing lactic acid by crystallizing a lactic acid salt from an aqueous lactic acid salt solution containing a formic acid salt, wherein the aqueous lactic acid salt solution contains a formic acid salt in an amount of not less than 7% by weight with respect to the lactic acid salt.

The "aqueous lactic acid salt solution" in the present invention means an aqueous solution containing a lactic acid salt. The aqueous solution is not limited as long as the solution is an aqueous solution containing a lactic acid salt, and may be a solution prepared by adding a lactic acid salt to water. Further, the aqueous lactic acid salt solution may be a lactic acid fermentation culture medium in cases where the lactic acid fermentation culture medium is produced by lactic acid fermentation culture by a lactic acid fermentation microorganism known to those skilled in the art and contains a lactic acid salt.

The lactic acid salt contained in the aqueous lactic acid salt solution in the present invention is not limited, and specific examples of the lactic acid salt include lithium lactate, sodium lactate, potassium lactate, calcium lactate, magnesium lactate, aluminum lactate and ammonium lactate. In cases where the lactic acid salt is calcium lactate or magnesium lactate, the solubility is relatively low and hence the recovery of the lactic acid salt in the crystallization operation is high, so that calcium lactate and magnesium lactate are preferred. Calcium lactate is more preferred.

The formic acid salt contained in the aqueous lactic acid salt solution in the present invention is not limited, and specific examples of the formic acid salt include sodium formate, potassium formate, lithium formate, calcium formate, magnesium formate, silicon formate, manganese formate, nickel formate, tin formate, iron formate, copper formate, cobalt formate, calcium/magnesium formate and ammonium formate. For example, in cases where the lactic acid salt is calcium lactate, the formic acid salt is preferably calcium formate, and in cases where the lactic acid salt is magnesium lactate, the formic acid salt is preferably magnesium formate. Thus, the formic acid salt preferably comprises the same metal ion as the metal ion in the lactic acid salt.

In cases where the aqueous lactic acid salt solution to be used is a lactic acid fermentation culture medium of a lactic acid fermentation microorganism, or a solution derived from a lactic acid fermentation culture medium, an alkaline substance, more specifically, a basic substance may be added for adjusting the pH of the fermentation culture medium. The alkaline substance to be added is not limited, and lithium hydroxide, sodium hydroxide, potassium hydroxide, calcium hydroxide, magnesium hydroxide, aluminum hydroxide, calcium carbonate, magnesium carbonate, calcium phosphate, magnesium phosphate, calcium oxide, magnesium oxide, calcium acetate, magnesium acetate or ammonia is preferably used. As a result, lithium lactate, sodium lactate, potassium lactate, calcium lactate, magnesium lactate, aluminum lactate or ammonium lactate is formed in the culture medium. As described above, in the present invention, the recovery of the lactic acid salt in the crystallization operation is high in cases where the lactic acid salt is calcium lactate or magnesium lactate. Therefore, as the alkaline substance to be added for the culture, calcium hydroxide, magnesium hydroxide, calcium carbonate, magnesium carbonate, calcium phosphate, magnesium phosphate, calcium oxide, magnesium oxide, calcium acetate or magnesium acetate is more preferably used. Calcium hydroxide or magnesium hydroxide is still more preferably used.

In the present invention, the term "aqueous lactic acid salt solution comprising a formic acid salt in an amount of not less than 7.0% by weight with respect to the lactic acid salt" means that a formic acid salt is contained in an amount of not less than 7.0% by weight with respect to the lactic acid salt in the aqueous lactic acid salt solution. In cases where, as a result of measurement of the amount of the lactic acid salt and the amount of the formic acid salt in the aqueous lactic acid salt solution, the amount of the formic acid salt was found to be less than 7% by weight with respect to the amount of the lactic acid salt, the formic acid salt is added to the aqueous lactic acid salt solution as appropriate. The amount of a lactic acid salt and the amount of a formic acid salt contained in an aqueous lactic acid salt solution can be quantified by high performance liquid chromatography (HPLC), and, based on the weights of the lactic acid salt and the formic acid salt contained in the aqueous lactic acid salt solution, the amount of the formic acid salt with respect to the amount of the lactic acid salt in the aqueous lactic acid salt solution can be calculated.

In cases where the amount of the formic acid salt with respect to the amount of the lactic acid salt in the aqueous lactic acid salt solution is less than 7.0% by weight, stability of supersaturation of the lactic acid salt is insufficient, and the effect of increasing the recovery of the lactic acid salt in the crystallization operation is low. The upper limit of the amount of the formic acid salt with respect to the amount of the lactic acid salt in the aqueous lactic acid salt solution is not limited as long as the ratio is within the range in which supersaturation of the lactic acid salt is stabilized, but, in cases where the ratio is higher than 40.0% by weight, the formic acid salt may be caught in lactic acid salt crystals recovered in the crystallization operation, and in such a case, the lactic acid salt crystals needs to be washed repeatedly for increasing the purity of the lactic acid salt. Therefore, the ratio is preferably 7.0 to 40.0% by weight, more preferably 7.2 to 30.0% by weight.

In the present invention, the term "subjecting an aqueous lactic acid salt solution to crystallization and recovering the lactic acid salt" means that an aqueous lactic acid salt solution in which a lactic acid salt is dissolved is cooled to obtain a lactic acid salt slurry, and the obtained lactic acid salt slurry is subjected to solid-liquid separation, followed by recovering the lactic acid salt precipitated thereby.

The temperature at which the aqueous lactic acid salt solution is cooled may be controlled such that the lactic acid salt precipitates due to a decreased saturation solubility. More specifically, the temperature is preferably not more than 30°C. As the temperature decreases, the recovery of the lactic acid salt can be increased. However, since a lower temperature requires more cooling energy, it is preferred to carry out the crystallization at a temperature condition of 10 to 30°C.

The lactic acid salt slurry obtained by crystallization is separated into crystals and the mother liquor by the operation of solid-liquid separation. The method of solid-liquid separation is not limited, and specific examples of the method include centrifugation, pressure filtration, suction filtration and cross-flow filtration. Since the mother liquor after solid-liquid separation contains the lactic acid salt at a concentration below the saturation solubility, the recovery of the lactic acid salt can be increased by subjecting the mother liquor again to the operation of crystallization. For example, since the lactic acid salt which could not be recovered by the operation of crystallization can be concentrated/recovered by passing the mother liquor through a reverse osmosis membrane, the lactic acid salt contained in the mother liquor can be recovered by subjecting the concentrate to the operation of crystallization.

On the other hand, since formic acid salts have high solubility, they do not precipitate as crystals at a normal crystallization temperature for lactic acid salts. Therefore, since almost 100% of the formic acid salt in the aqueous lactic acid salt solution is contained in the mother liquor side after the lactic acid salt crystallization, recycling of the mother liquor allows production of a certain level of the effect to increase the recovery of the lactic acid salt also in a continuous operation of crystallization.

In some cases, formic acid and other impurities, and, especially in cases where the lactic acid salt is derived from a fermentation culture medium of a microorganism, components of the fermentation culture medium and by-products, are attached to the crystals after the solid-liquid separation. Therefore, a highly pure lactic acid salt can be obtained by washing the crystals. The washing of the crystals may be carried out either during the solid-liquid separation or after the solid-liquid separation. As the washing agent, pure water may be used, but in cases of washing with pure water, a part of the lactic acid salt may be dissolved, resulting in low recovery. By performing washing using a saturated aqueous solution of the same lactic acid salt as the lactic acid salt to be recovered, the decrease in the recovery can be suppressed. Further, after washing of the crystals with pure water or a saturated aqueous lactic acid salt solution, the washing liquid may be subjected to crystallization again to suppress the decrease in the recovery of the lactic acid salt.

The lactic acid salt concentration in the aqueous lactic acid salt solution to be subjected to the operation of crystallization is not limited, and the concentration is preferably 10.0 to 30.0% by weight. In cases where the concentration is not less than 10.0% by weight, the recovery after crystallization can be increased, but in cases where the concentration is higher than 30.0% by weight, uniform stirring in the crystallization tank may be disturbed by slurrying, causing a problem in operability. In cases where the lactic acid salt concentration in the aqueous lactic acid salt solution is less than 10.0% by weight, the crystallization is preferably carried out after increasing the lactic acid salt concentration to not less than 10.0% by weight by the operation of concentration.

The liquid temperature of the aqueous lactic acid salt solution subjected to the crystallization operation is not limited as long as the temperature does not cause loss of the lactic acid salt before the crystallization operation, that is, the temperature does not cause precipitation of the lactic acid salt. The liquid temperature is adjusted to preferably not less than 35°C, more preferably not less than 40°C.

Examples of the method for concentrating the aqueous lactic acid salt solution include a method by evaporation of water using a concentration apparatus represented by an evaporator under heat and/or reduced pressure, and a method by increasing the lactic acid salt concentration using a reverse osmosis membrane. In view of reduction of the energy required for concentration, the concentration method using a reverse osmosis membrane is preferred. Concentration of the aqueous lactic acid salt solution using a reverse osmosis membrane may be carried out according to the method described in JP 2010-57389 A.

The liquid temperature during the concentration of the aqueous lactic acid salt solution using a reverse osmosis membrane is not limited, and is adjusted to preferably 30 to 60°C, more preferably 35 to 55°C. The concentration with a reverse osmosis membrane may be usually carried out to a concentration at which the solid content does not precipitate. Since the saturation solubility of a lactic acid salt increases as the temperature increases, a concentrate at high concentration can be obtained without causing precipitation of the lactic acid salt if the temperature of the culture medium containing the lactic acid salt is not less than 30°C. On the other hand, in cases where the temperature during the operation of passing the solution through a reverse osmosis membrane is higher than 60°C, the permeability may gradually decrease due to structural changes in the reverse osmosis membrane, causing a problem in a long-term filtration operation with the reverse osmosis membrane.

When the aqueous solution containing a lactic acid salt is passed through a reverse osmosis membrane, the operational pressure is preferably within the range of 1 to 8 MPa since a pressure lower than 1 MPa results in a decreased membrane permeation rate, while a pressure higher than 8 MPa damages the membrane. In cases where the filtration pressure is within the range of 1 to 7 MPa, the membrane permeation flux is high, so that efficient permeation of water is possible and there is less possibility of damaging the membrane, which is more preferred. The filtration pressure is still more preferably within the range of 2 to 6 MPa.

Examples of the membrane material of the reverse osmosis membrane which may be used in the present invention include macromolecular materials which are commercially generally available, such as cellulose acetate polymers, polyamides, polyesters, polyimides, vinyl polymers and polysulfones. The membrane is not restricted to a membrane constituted by only one of the materials, and may be a membrane comprising a plurality of the membrane materials. In terms of the structure of the membrane, the membrane may be either an asymmetric membrane which has a dense layer on at least one side and micropores having pore sizes that gradually increase in the direction from the dense layer toward the inside of the membrane or the other side of the membrane, or a composite membrane which has a very thin functional layer formed by another material on the dense layer of an asymmetric membrane.

Examples of the reverse osmosis membrane preferably used in the present invention include a composite membrane comprising a cellulose acetate polymer as a functional layer (which may be hereinafter referred to as cellulose acetate reverse osmosis membrane) and a composite membrane comprising a polyamide as a functional layer (which may be hereinafter referred to as polyamide reverse osmosis membrane). Examples of the cellulose acetate polymer herein include polymers prepared with organic acid esters of cellulose such as cellulose acetate, cellulose diacetate, cellulose triacetate, cellulose propionate and cellulose butyrate, which may be used individually, as a mixture, or as a mixed ester. Examples of the polyamide include linear polymers and cross-linked polymers constituted by aliphatic and/or aromatic diamine monomers. Since polyamide reverse osmosis membranes show especially high blocking rates for lactic acid salts and high recovery of lactic acid salts, a polyamide reverse osmosis membrane is preferably used in the present invention.

The form of the membrane may be appropriately selected, and examples of the membrane which may be used include flat membranes, spiral-wound membranes and hollow fiber membranes.

Specific examples of a reverse osmosis membrane preferably used in the present invention include UTC-70, SU-710, SU-720, SU-720F, SU-710L, SU-720L, SU-720LF, SU-720R, SU-710P, SU-720P, SU-810, SU-820, SU-820L, SU-820FA, SU-610, SU-620, TM800, TM800C, TM800A, TM800H, TM800E and TM800L, which are polyamide reverse osmosis membranes manufactured by TORAY INDUSTRIES, INC.; SC-L100R, SC-L200R, SC-1100, SC-1200, SC-2100, SC-2200, SC-3100, SC-3200, SC-8100 and SC-8200, which are cellulose acetate reverse osmosis membranes manufactured by TORAY INDUSTRIES, INC.; NTR-759HR, NTR-729HF, NTR-70SWC, ES10-D, ES20-D, ES20-U, ES15-D, ES 15-U and LF10-D, which are manufactured by Nitto Denko Corporation; RO98pHt, RO99, HR98PP, CE4040C-30D, NF99 and NF99HF, which are manufactured by Alfa-Laval; A Series, GE Sepa, OSMO BEV NF Series, HL Series, Duraslick Series, MUNI RO Series, MUNI NF Series, MUNI RO LE Series, Duratherm RO HF Series, CK Series, DK Series, Seasoft Series, Duratherm RO HF Series, Duratherm HWS Series, PRO RO Series and PRO RO LE Series, which are manufactured by GE; BLF series, BLR series and BE series, which are manufactured by SAEHAN CSM; SelRO Series, which is manufactured by KOCH; and BW30-4040, TW30-4040; XLE-4040, LP-4040, LE-4040, SW30-4040, SW30HRLE-4040, NF45, NF90, NF200 and NF400, which are manufactured by Filmtec.

### EXAMPLES

The present invention is described below by way of Examples in more detail, but the present invention is not limited by the Examples below.

### Reference Example 1

### Measurement of Saturation Solubility of Aqueous Calcium Lactate Solution Containing Calcium Formate

To 50 g of calcium lactate pentahydrate (manufactured by Sigma-Aldrich), 100 g of pure water was added, to prepare 23.6% by weight of aqueous calcium lactate anhydride solution. Further, aqueous calcium lactate solutions each containing calcium formate (manufactured by Sigma-Aldrich) in an amount of 0% by weight, 2.5% by weight, 7.2% by weight, 14.5% by weight, or 25.0% by weight with respect to calcium lactate anhydride were prepared to provide test solutions. With incubation at 20°C, 30°C, 40°C or 50°C, each prepared test solution was stirred at 400 rpm. The calcium lactate slurry after incubation for 1, 3 or 6 hour(s) at each temperature was filtered through a 0.2-µm filter, and the concentration of calcium lactate anhydride in the filtrate was measured to determine the saturation solubility. The calcium lactate concentration and the calcium formate concentration in the aqueous calcium lactate solution were measured using a high performance liquid chromatography (manufactured by Shimadzu Corporation) under the following conditions.
Column: Shim-Pack SPR-H (manufactured by Shimadzu Corporation)
Mobile phase: 5 mM p-toluenesulfonic acid (flow rate, 0.8 mL/min.)
Reaction liquid: 5 mM p-toluenesulfonic acid, 20 mM Bis-Tris, 0.1 mM EDTA-2Na (flow rate, 0.8 mL/min.)
Detection method: electric conductivity
Temperature: 45 °C

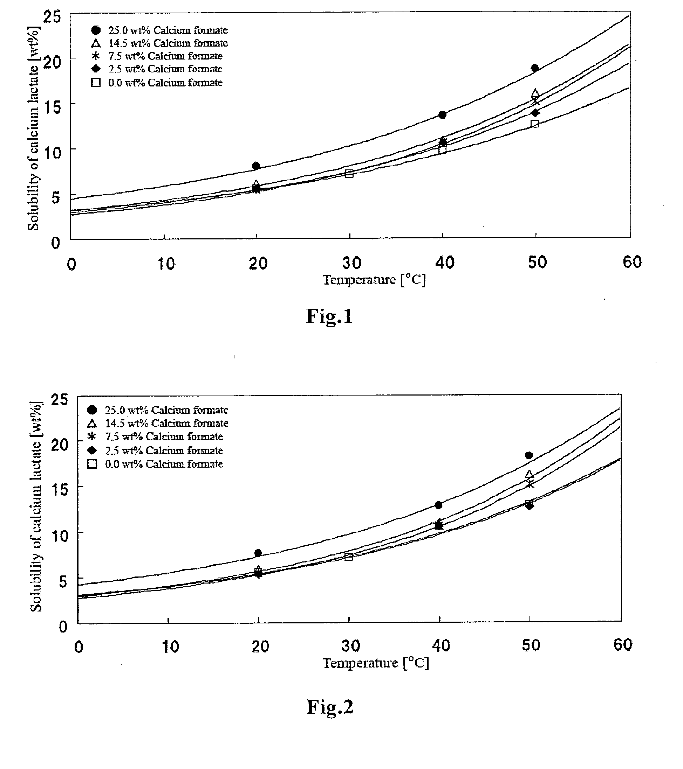

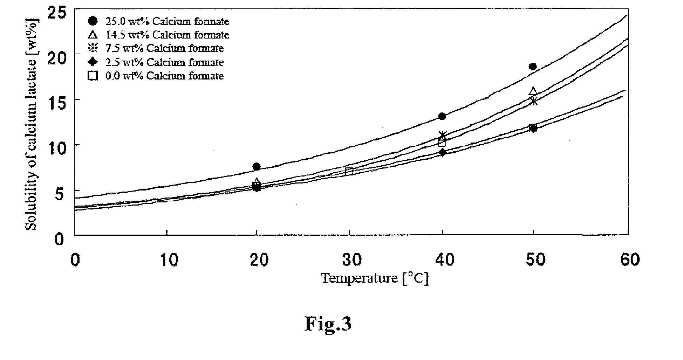

The results were as shown in Figs. 1 to 3. In cases where formic acid was contained in an amount larger than 7.2% by weight with respect to calcium lactate, the solubility did not decrease even after 6 hours of incubation, so that stabilization of supersaturation was indicated. That is, it was shown that utilization of the stability of supersaturation allows suppression of precipitation of crystals, and hence concentration of the solution to a high concentration, so that the recovery by the crystallization operation can be increased.

### Examples 1 and 2

### Crystallization with Aqueous Calcium Lactate Solution Containing 7.5% by Weight of Calcium Formate

To 100 g of calcium lactate pentahydrate (manufactured by Sigma-Aldrich), 250 g of pure water and 4.5 g of calcium formate (manufactured by Sigma-Aldrich) were added, to prepare 20.0% by weight of aqueous calcium lactate solution. The solution was then stirred at 50°C at 400 rpm for 2 hours, and solid-liquid separation was carried out by suction filtration with Qualitative Filter Paper No. 2 (manufactured by ADVANTEC) to remove undissolved calcium lactate, followed by recovering the mother liquor. The calcium lactate concentration and the calcium formate concentration in the recovered mother liquor were measured by high performance liquid chromatography as in Reference Example 1. As a result, the calcium lactate concentration in the recovered mother liquor was 15.1 % by weight, and the amount of calcium formate with respect to calcium lactate was 7.5% by weight. The recovered mother liquor as a test solution was divided into two aliquots, and each aliquot was cooled to 20°C or 30°C, followed by stirring at 400 rpm for 2 hours. The precipitated slurry was subjected to solid-liquid separation by suction filtration with Qualitative Filter Paper No. 2 (manufactured by ADVANTEC) into wet crystals and the mother liquor. The amount of calcium lactate in the wet crystals was measured by high performance liquid chromatography as in Reference Example 1, and the recovery of calcium lactate was calculated according to the method of Equation 1. The results are shown in Table 1 (a) and (b).

Recovery of calcium lactate (%) = 100 x amount of calcium lactate in wet crystals (g) / amount of calcium lactate in test solution (g) ... (Equation 1).

### Examples 3 and 4

### Crystallization with Aqueous Calcium Lactate Solution Containing 14.5% by Weight of Calcium Formate with Respect to Calcium Lactate

To 100 g of calcium lactate pentahydrate (manufactured by Sigma-Aldrich), 240 g of pure water and 10 g of calcium formate (manufactured by Sigma-Aldrich) were added, to prepare 20.2% by weight of aqueous calcium lactate solution. The solution was then stirred at 50°C at 400 rpm for 2 hours, and solid-liquid separation was carried out by suction filtration with Qualitative Filter Paper No. 2 (manufactured by ADVANTEC) to remove undissolved calcium lactate, followed by recovering the mother liquor. The calcium lactate concentration and the calcium formate concentration in the recovered mother liquor were measured by high performance liquid chromatography as in Reference Example 1. As a result, the calcium lactate concentration in the recovered mother liquor was 15.5% by weight, and the amount of calcium formate with respect to calcium lactate was 14.5% by weight. The recovered mother liquor as a test solution was subjected to crystallization of calcium lactate followed by solid-liquid separation in the same manner as in Examples 1 and 2, and the recovery of calcium lactate was calculated according to the method of Equation 1. The results are shown in Table 1 (c) and (d).

### Examples 5 and 6

### Crystallization with Aqueous Calcium Lactate Solution Containing 25% by Weight of Calcium Formate with Respect to Calcium Lactate

To 100 g of calcium lactate pentahydrate (manufactured by Sigma-Aldrich), 230 g of pure water and 17.0 g of calcium formate (manufactured by Sigma-Aldrich) were added, to prepare 20.4% by weight of aqueous calcium lactate solution. The solution was then stirred at 50°C at 400 rpm for 2 hours, and solid-liquid separation was carried out by suction filtration with Qualitative Filter Paper No. 2 (manufactured by ADVANTEC) to remove undissolved calcium lactate, followed by recovering the mother liquor. The calcium lactate concentration and the calcium formate concentration in the recovered mother liquor were measured by high performance liquid chromatography as in Reference Example 1. As a result, the calcium lactate concentration in the recovered mother liquor was 19.5% by weight, and the amount of calcium formate with respect to calcium lactate was 25.0% by weight. The recovered mother liquor as a test solution was subjected to crystallization of calcium lactate followed by solid-liquid separation in the same manner as in Examples 1 and 2, and the recovery of calcium lactate was calculated according to the method of Equation 1. The results are shown in Table 1 (e) and (f).

### Comparative Examples 1 and 2

### Crystallization with Aqueous Calcium Lactate Solution Containing 0% by Weight of Calcium Formate with Respect to Calcium Lactate

To 100 g of calcium lactate pentahydrate (manufactured by Sigma-Aldrich), 254 g of pure water was added, to prepare 20.0% by weight of aqueous calcium lactate solution. The solution was then stirred at 50°C at 400 rpm for 2 hours, and solid-liquid separation was carried out by suction filtration with Qualitative Filter Paper No. 2 (manufactured by ADVANTEC) to remove undissolved calcium lactate, followed by recovering the mother liquor. The calcium lactate concentration and the calcium formate concentration in the recovered mother liquor were measured by high performance liquid chromatography as in Reference Example 1. As a result, the calcium lactate concentration in the recovered mother liquor was 12.5% by weight, and the amount of calcium formate with respect to calcium lactate was 0% by weight. The recovered mother liquor as a test solution was subjected to crystallization of calcium lactate followed by solid-liquid separation in the same manner as in Examples 1 and 2, and the recovery of calcium lactate was calculated according to the method of Equation 1. The results are shown in Table 1 (g) and (h).

### Comparative Examples 3 and 4

### Crystallization with Aqueous Calcium Lactate Solution Containing 2.5% by Weight of Calcium Formate with Respect to Calcium Lactate

To 100 g of calcium lactate pentahydrate (manufactured by Sigma-Aldrich), 252 g of pure water and 1.7 g of calcium formate (manufactured by Sigma-Aldrich) were added, to prepare 20.0% by weight of aqueous calcium lactate solution. The solution was then stirred at 50°C at 400 rpm for 2 hours, and solid-liquid separation was carried out by suction filtration with Qualitative Filter Paper No. 2 (manufactured by ADVANTEC) to remove undissolved calcium lactate, followed by recovering the mother liquor. The calcium lactate concentration and the calcium formate concentration in the recovered mother liquor were measured by high performance liquid chromatography as in Reference Example 1. As a result, the calcium lactate concentration in the recovered mother liquor was 12.5% by weight, and the amount of calcium formate with respect to calcium lactate was 2.5% by weight. The recovered mother liquor as a test solution was subjected to crystallization of calcium lactate followed by solid-liquid separation in the same manner as in Examples 1 and 2, and the recovery of calcium lactate was calculated according to the method of Equation 1. The results are shown in Table 1 (i) and (j).

**[Table 1]**

| | | Test solution | | Crystallization operation | |
|---|---|---|---|---|---|
| | | Calcium lactate concentration (% by weight) | Amount of calcium formate with respect to calcium lactate (% by weight) | Crystallization temperature (°C) | Recovery of calcium lactate (%) |
| a | Example 1 | 15.1 | 7.5 | 30 | 45 |
| b | Example 2 | 15.1 | 7.5 | 20 | 60 |
| c | Example 3 | 15.5 | 14.5 | 30 | 53 |
| d | Example 4 | 15.5 | 14.5 | 20 | 65 |
| e | Example 5 | 19.5 | 25.0 | 30 | 54 |
| f | Example 6 | 19.5 | 25.0 | 20 | 63 |
| g | Comparative Example 1 | 12.5 | 0.0 | 30 | 34 |
| h | Comparative Example 2 | 12.5 | 0.0 | 20 | 43 |
| i | Comparative Example 3 | 12.5 | 2.5 | 30 | 32 |
| J | Comparative Example 4 | 12.5 | 2.5 | 20 | 44 |

As shown in Examples 1 to 6 in Table 1, it was shown that, as the concentration of calcium formate with respect to the concentration of calcium lactate increases, the concentration of calcium lactate in the test solution increases, and the recovery of calcium lactate by the crystallization operation increases. On the other hand, as shown in Comparative Examples 1 to 4, in the cases where the concentration of calcium formate with respect to the concentration of calcium lactate was 0 or 2.5% by weight, the concentration of calcium lactate in the test solution did not change, so that it was shown that the recovery of calcium lactate by the crystallization operation does not change.

### Comparative Examples 5 and 6

### Crystallization with Aqueous Calcium Lactate Solution Containing 14.5% by Weight of Calcium Acetate with Respect to Calcium Lactate

To 100 g of calcium lactate pentahydrate (manufactured by Sigma-Aldrich), 240 g of pure water and 10 g of calcium acetate (manufactured by Sigma-Aldrich) were added, to prepare 20.2% by weight of aqueous calcium lactate solution. The solution was then stirred at 50°C at 400 rpm for 2 hours, and solid-liquid separation was carried out by suction filtration with Qualitative Filter Paper No. 2 (manufactured by ADVANTEC) to remove undissolved calcium lactate, followed by recovering the mother liquor. The calcium lactate concentration and the calcium acetate concentration in the recovered mother liquor were measured by high performance liquid chromatography as in Reference Example 1. As a result, the calcium lactate concentration in the recovered mother liquor was 12.4% by weight, and the amount of calcium acetate with respect to calcium lactate was 14.5% by weight. The recovered mother liquor as a test solution was subjected to crystallization of calcium lactate followed by solid-liquid separation in the same manner as in Examples 1 and 2, and the recovery of calcium lactate was calculated according to the method of Equation 1. The results were as shown in Table 2. Crystallization of calcium lactate containing calcium acetate did not cause any change in the calcium lactate concentration in the test solution, and it was therefore shown that calcium acetate is not effective for increasing the recovery of calcium lactate.

**[Table 2]**

| | | Test solution | | Crystallization operation | |
|---|---|---|---|---|---|
| | | Calcium lactate concentration (% by weight) | Amount of calcium acetate with respect to calcium lactate (% by weight) | Crystallization temperature (°C) | Recovery of calcium lactate (%) |
| a | Comparative Example 5 | 12.4 | 14.5 | 30 | 32 |
| b | Comparative Example 6 | 12.4 | 14.5 | 20 | 43 |

### Reference Example 2

### Production of L-Lactic Acid Fermentation Culture Medium Using L-Lactic Acid Bacterium

As an L-lactic acid bacterium, the *Lactobacillus casei* NRIC1941 strain was selected (hereinafter referred to as LC strain). The LC strain was subjected to static culture in a test tube containing 5 mL of a nitrogen-purged pre-pre-preculture medium (100 g/L cane juice, 10 g/L yeast extract) for 24 hours at a temperature of 30°C (pre-pre-preculture). The medium was autoclaved (121°C, 15 minutes) before use. The obtained pre-pre-preculture was inoculated to 50 mL of the same nitrogen-purged medium, and the resultant was subjected to static culture for 24 hours at a temperature of 30°C (pre-preculture). The obtained pre-preculture was inoculated to 1 L of the same nitrogen-purged medium, and subjected to static culture for 24 hours at a temperature of 30°C (preculture). The obtained preculture was inoculated to the same medium, and cultured with shaking at 30°C at 300 rpm while the pH was adjusted by addition of calcium hydroxide until the end of the culture. As a result of the pH adjustment, calcium lactate and calcium formate were produced in the culture medium. The fermentation test was carried out for 90 hours, and the concentration of calcium lactate and the concentration of calcium formate contained in the fermentation culture medium were measured. As a result, the concentration of calcium lactate was 4.5% by weight, and the amount of calcium formate with respect to calcium lactate was 2.7% by weight.

### Examples 7 and 8

### Crystallization of Calcium Lactate from L-Lactic Acid Fermentation Culture Medium Obtained Using LC Strain

Through a microfiltration membrane ("Microza", manufactured by Asahi Kasei Chemicals Corporation), 30 L of the lactic acid fermentation culture medium obtained in Reference Example 2 was filtered to remove bacterial cells, and 120 g of calcium formate was added to the obtained clear filtrate, followed by incubating of the resulting mixture at 50°C and concentrating the mixture with a spiral-wound 4-inch reverse osmosis membrane element ("TM-810", manufactured by TORAY INDUSTRIES, INC.) such that the concentration of calcium lactate became 15% by weight. The calcium lactate concentration and the calcium formate concentration in the recovered concentrate were measured by high performance liquid chromatography as in Reference Example 1. As a result, the calcium lactate concentration in the recovered concentrate was 15.0% by weight, and the amount of calcium formate with respect to calcium lactate was 10.5% by weight. The recovered concentrate as a test solution was subjected to crystallization of calcium lactate followed by solid-liquid separation in the same manner as in Examples 1 and 2, and the recovery of calcium lactate was calculated according to the method of Equation 1. The results are shown in Table 3 (a) and (b).

### Reference Example 3

### Production of D-lactic Acid Fermentation Culture Medium Using D-lactic Acid Bacterium

As a D-lactic acid bacterium, the *Sporolactobacillus laevolacticus* ATCC23492 strain was selected (hereinafter referred to as SL strain). The SL strain was subjected to static culture in a test tube containing 5 mL of a nitrogen-purged main culture medium (5 g/L calcium carbonate, 10 g/L polypeptone, 3 g/L yeast extract, 0.5 g/L potassium phosphate, 0.5 g/L potassium dihydrogen phosphate, 0.3 g/L magnesium sulfate heptahydrate, 0.01 g/L sodium chloride) for 24 hours at a temperature of 30°C (preculture). The obtained preculture was inoculated to the same medium, and cultured with shaking at 37°C at 120 rpm while the pH was adjusted by addition of calcium hydroxide until the end of the culture. As a result of the ppH adjustment, calcium lactate and calcium formate were produced in the culture medium. The fermentation test was carried out for 160 hours, and the concentration of calcium lactate and the concentration of calcium formate contained in the fermentation culture medium were measured. As a result, the concentration of calcium lactate was 6.0% by weight, and the amount of calcium formate with respect to calcium lactate was 0.8% by weight.

### Examples 9 and 10

### Crystallization of Calcium Lactate from D-lactic Acid Fermentation Culture Medium Obtained Using SL Strain

Through a microfiltration membrane ("Microza", manufactured by Asahi Kasei Chemicals Corporation), 30 L of the lactic acid fermentation culture medium obtained in Reference Example 3 was filtered to remove bacterial cells, and 276 g of calcium formate was added to the obtained clear filtrate, followed by incubating of the resulting mixture at 50°C and concentrating the mixture with a spiral-wound 4-inch reverse osmosis membrane element ("TM-810", manufactured by TORAY INDUSTRIES, INC.) such that the concentration of calcium lactate became 15.0% by weight. The calcium lactate concentration and the calcium formate concentration in the recovered concentrate were measured by high performance liquid chromatography as in Reference Example 1. As a result, the calcium lactate concentration in the recovered concentrate was 15.0% by weight, and the amount of calcium formate with respect to calcium lactate was 10.0% by weight. The recovered concentrate as a test solution was subjected to crystallization of calcium lactate followed by solid-liquid separation in the same manner as in Examples 1 and 2, and the recovery of calcium lactate was calculated according to the method of Equation 1. The results are shown in Table 3 (c) and (d).

### Reference Example 4

### Production of L-Lactic Acid Fermentation Culture Medium Using L-Lactic Acid Fermentation Yeast

Using the L-lactic acid fermentation yeast HI003 strain (hereinafter referred to as HI003 strain) described in WO 2009/099044 and a raw sugar medium (70 g/L "Yutosei" (manufactured by MUSO Co., Ltd.), 1.5 g/L ammonium sulfate), a batch fermentation test was carried out. The medium was autoclaved (121°C, 15 minutes) before use. Evaluation of the concentration of lactic acid, which is the product, was carried out using HPLC shown in Reference Example 1, and the glucose concentration was measured using "Glucose Test Wako C" (manufactured by Wako Pure Chemical Industries, Ltd.). The operating conditions in Reference Example 2 were as shown below.

Reactor capacity (amount of lactic acid fermentation medium), 30 (L); temperature adjustment, 32 (°C); reactor ventilation volume, 0.1 (L/min.); reactor stirring speed, 200 (rpm); pH adjustment, adjusted to pH 6.5 with 1 N calcium hydroxide.

First, the HI003 strain was cultured in 5 ml of the raw sugar medium in a test tube overnight with shaking (pre-preculture). The pre-preculture was inoculated to 100 ml of a fresh raw sugar medium, and culture was performed in a 500-ml Sakaguchi flask for 24 hours with shaking (preculture). While the temperature was adjusted, and the pH was adjusted with calcium hydroxide, fermentation culture was performed. As a result of the pH adjustment, calcium lactate and calcium formate were produced in the culture medium. As a result of the culture for 50 hours, the concentration of calcium lactate was 4.5% by weight, and calcium formate could not be detected.

### Examples 11 and 12

### Crystallization of Calcium Lactate from L-Lactic Acid Fermentation Culture Medium Obtained Using L-Lactic Acid Fermentation Yeast

Through a microfiltration membrane ("Microza", manufactured by Asahi Kasei Chemicals Corporation), 30 L of the lactic acid fermentation culture medium obtained in Reference Example 4 was filtered to remove bacterial cells, and 190 g of calcium formate was added to the obtained clear filtrate, followed by incubating of the resulting mixture at 50°C and concentrating the mixture with a spiral-wound 4-inch reverse osmosis membrane element ("TM-810, manufactured by TORAY INDUSTRIES, INC.) such that the concentration of calcium lactate became 15.0% by weight (the concentration of calcium formate with respect to the concentration of calcium lactate was 10% by weight). The calcium lactate concentration and the calcium formate concentration in the recovered concentrate were measured by high performance liquid chromatography as in Reference Example 1. As a result, the calcium lactate concentration in the recovered concentrate was 15.0% by weight, and the amount of calcium formate with respect to calcium lactate was 10.2% by weight. The recovered concentrate as a test solution was subjected to crystallization of calcium lactate followed by solid-liquid separation in the same manner as in Examples 1 and 2, and the recovery of calcium lactate was calculated according to the method of Equation 1. The results are shown in Table 3 (e) and (f).

### Comparative Examples 7 and 8

### Crystallization of Calcium Lactate from L-Lactic Acid Fermentation Culture Medium Obtained Using LC Strain

Through a microfiltration membrane ("Microza", manufactured by Asahi Kasei Chemicals Corporation), 30 L of the L-lactic acid fermentation culture medium obtained in Reference Example 2 was filtered to remove bacterial cells, and incubation was carried out at 50°C without addition of calcium formate, followed by concentration with a spiral-wound 4-inch reverse osmosis membrane element ("TM-810, manufactured by TORAY INDUSTRIES, INC.). However, when the concentration of calcium lactate reached 12.8% by weight, precipitation of calcium lactate was found, so that the concentration was terminated. While the recovered concentrate was incubated at 50°C, the concentrate was filtered by suction filtration through Qualitative Filter Paper No. 2 (manufactured by ADVANTEC) in order to remove the precipitated calcium lactate crystals. The calcium lactate concentration and the calcium formate concentration in the recovered concentrate were measured by high performance liquid chromatography as in Reference Example 1. As a result, the calcium lactate concentration in the recovered concentrate was 12.8% by weight, and the amount of calcium formate with respect to calcium lactate was 0.3% by weight. The recovered concentrate as a test solution was subjected to crystallization of calcium lactate followed by solid-liquid separation in the same manner as in Examples 1 and 2, and the recovery of calcium lactate was calculated according to the method of Equation 1. The results are shown in Table 3 (g) and (h).

### Comparative Examples 9 and 10

### Crystallization of Calcium Lactate from D-lactic Acid Fermentation Culture Medium Obtained Using SL Strain

As in Comparative Examples 7 and 8, 30 L of the D-lactic acid fermentation culture medium obtained in Reference Example 3 was concentrated without addition of calcium formate. However, when the concentration of calcium lactate reached 12.5% by weight, precipitation of calcium lactate was found, so that the concentration was terminated. While the recovered concentrate was incubated at 50°C, the concentrate was filtered by suction filtration through Qualitative Filter Paper No. 2 (manufactured by ADVANTEC) in order to remove the precipitated calcium lactate crystals. The calcium lactate concentration and the calcium formate concentration in the recovered concentrate were measured by high performance liquid chromatography as in Reference Example 1. As a result, the calcium lactate concentration in the recovered concentrate was 12.4% by weight, and the amount of calcium formate with respect to calcium lactate was 0.4% by weight. The recovered concentrate as a test solution was subjected to crystallization of calcium lactate followed by solid-liquid separation in the same manner as in Examples 1 and 2, and the recovery of calcium lactate was calculated according to the method of Equation 1. The results are shown in Table 3 (i) and (j).

### Comparative Examples 11 and 12

### Crystallization of Calcium Lactate from L-Lactic Acid Fermentation Culture Medium Obtained Using L-Lactic Acid Fermentation Yeast

As in Comparative Examples 7 and 8, 30 L of the L-lactic acid fermentation culture medium obtained in Reference Example 4 was concentrated without addition of calcium formate. However, when the concentration of calcium lactate reached 12.0% by weight, precipitation of calcium lactate was found, so that the concentration was terminated. While the recovered concentrate was incubated at 50°C, the concentrate was filtered by suction filtration through Qualitative Filter Paper No. 2 (manufactured by ADVANTEC) in order to remove the precipitated calcium lactate crystals. The calcium lactate concentration and the calcium formate concentration in the recovered concentrate were measured by high performance liquid chromatography as in Reference Example 1. As a result, the calcium lactate concentration in the recovered concentrate was 12.1% by weight, and the amount of calcium formate with respect to calcium lactate was 0% by weight. The recovered concentrate as a test solution was subjected to crystallization of calcium lactate followed by solid-liquid separation in the same manner as in Examples 1 and 2, and the recovery of calcium lactate was calculated according to the method of Equation 1. The results are shown in Table 3 (k) and (l).

**[Table 3]**

| | | Test solution | | Crystallization operation | |
|---|---|---|---|---|---|
| | | Calcium lactate concentration (% by weight) | Amount of calcium formate with respect to calcium lactate (% by weight) | Crystallization temperature (°C) | Recovery of calcium lactate (%) |
| a | Example 7 | 15.0 | 10.5 | 30 | 53 |
| b | Example 8 | 15.0 | 10.5 | 20 | 63 |
| c | Example 9 | 15.0 | 10.0 | 30 | 51 |
| d | Example 10 | 15.0 | 10.0 | 20 | 63 |
| e | Example 11 | 15.0 | 10.2 | 30 | 51 |
| f | Example 12 | 15.0 | 10.2 | 20 | 62 |
| g | Comparative Example 7 | 12.8 | 0.3 | 30 | 36 |
| h | Comparative Example 8 | 12.8 | 0.3 | 20 | 42 |
| i | Comparative Example 9 | 12.4 | 0.4 | 30 | 35 |
| J | Comparative Example 10 | 12.4 | 0.4 | 20 | 40 |
| k | Comparative Example 11 | 12.1 | 0.0 | 30 | 37 |
| l | Comparative Example 12 | 12.1 | 0.0 | 20 | 44 |

As shown in Table 3, it was shown that, also in cases where calcium lactate is crystallized from a lactic acid fermentation culture medium, the recovery of calcium lactate after the crystallization operation is high if not less than 7% by weight of calcium formate with respect to calcium lactate is contained in the lactic acid fermentation culture medium. Further, it was shown that, in cases where no formic acid salt is contained in the lactic acid fermentation culture medium produced by a microorganism, addition of a formic acid salt thereto before the concentration can increase the recovery of calcium lactate after the crystallization operation.

### INDUSTRIAL APPLICABILITY

The lactic acid salt obtained by the present invention can be used not only for uses such as food and pharmaceuticals, but also as a raw material for biodegradable plastics.

## Claims

1. A method for producing a lactic acid salt, said method comprising the step of subjecting an aqueous lactic acid salt solution comprising a formic acid salt in an amount of not less than 7.0% by weight with respect to said lactic acid salt to crystallization, and recovering said lactic acid salt.

2. The method for producing a lactic acid salt according to claim 1, wherein said aqueous lactic acid salt solution is an aqueous lactic acid salt solution comprising a formic acid salt in an amount of 7.0 to 40.0% by weight with respect to said lactic acid salt.

3. The method for producing a lactic acid salt according to claim 1 or 2, wherein said lactic acid salt is calcium lactate or magnesium lactate.

4. The method for producing a lactic acid salt according to any one of claims 1 to 3, wherein the lactic acid salt concentration in said aqueous lactic acid salt solution is 10.0 to 30.0% by weight.

5. The method for producing a lactic acid salt according to any one of claims 1 to 4, wherein crystallization of said lactic acid salt is carried out at a temperature of not more than 30°C.

6. The method for producing a lactic acid salt according to any one of claims 1 to 5, wherein a concentrate obtained by passing said aqueous lactic acid salt solution through a reverse osmosis membrane at 30 to 60°C is subjected to crystallization.
